# EUROPÄISCHE PATENTANMELDUNG

(11) **EP 4 557 760 A1**
(43) Veröffentlichungstag der Anmeldung: **21.05.2025**
(21) Anmeldenummer: 24212861.9
(22) Anmeldetag: 14.11.2024
(51) Int. Cl.: H04Q 9/00, G01N 33/38

(54) **VERFAHREN ZUR ÜBERWACHUNG EINES BAUWERKES**

(30) Priorität: 14.11.2023 DE 102023131633
(71) Anmelder: Koch Carbon Consulting GmbH, 57223 Kreuztal (DE)
(72) Erfinder: Koch, Detlef, 57223 Kreuztal (DE)
(74) Vertreter: Tergau & Walkenhorst Intellectual Property GmbH

(57) **Zusammenfassung**

Ein Verfahren zur Überwachung eines Bauwerkes aus Stahlbeton und ein Stahlbetonüberwachungssystem (1) mit einer Mehrzahl von Endgeräten (2), wobei ein Endgerät (2) mindestens ein Sensorelement umfasst oder mit mindestens einem Sensorelement signaltechnisch verbunden ist und mit mindestens einem Gateway (4), wobei die Endgeräte (2) signaltechnisch direkt oder indirekt mit dem Gateway (4) verbunden sind und das Gateway (4) zur Kommunikation mit einer Cloud (6) oder einer externen Auswerteeinheit ausgebildet ist, soll besonders einfach, umfassend und energieeffizient den Zustand eines Bauwerkes erfassen. Dazu wird die Ansteuerung der Sensorelemente und/oder die Auswertung der gemessenen Sensordaten durch die Sensorelemente durch die Cloud (6) oder die externe Auswerteeinheit initiiert bzw. durchgeführt.

## Beschreibung

Die Erfindung betrifft ein Verfahren zur Überwachung eines Bauwerkes aus Stahlbeton und ein Stahlbetonbauwerküberwachungssystem mit einer Mehrzahl von Endgeräten, wobei ein Endgerät mindestens ein Sensorelement umfasst oder mit mindestens einem Sensorelement signaltechnisch verbunden ist und mindestens einem Gateway, wobei die Endgeräte signaltechnisch direkt oder indirekt mit dem Gateway verbunden sind und das Gateway zur Kommunikation mit einer Cloud oder einer externen Auswerteeinheit ausgebildet ist.

Bauwerke aus Stahlbeton sind integraler Bestandteil der Infrastruktur in fast allen Ländern der Welt. Neben Wohn- und Arbeitsgebäuden sind auch viele befahrene Bauwerke aus Stahlbeton gebaut, z. B. Parkhäuser, Garagen, Autobahnen, Brücken, Tunnel usw. Eine Großzahl dieser Bauwerke wird 50 bis 100 Jahre (und teilweise noch länger) genutzt. Allerdings setzen neben der mechanischen Beanspruchung auch viele Umwelteinflüsse, wie beispielsweise Winde, Tausalze oder chemische Belastungen den Stahlbetonbauwerken zu. Die Tausalze sind in der Regel chloridhaltig. Es entstehen daher in Verbindung mit Wasser Lösungen, die Korrosion in den Bauwerken auslösen. Bei vielen Bauwerken müssen deshalb bereits nach 20-25 Jahren substanzielle, kostenintensive Instandsetzungsarbeiten an der Bewehrung durchgeführt werden.

Dazu wird üblicherweise der kontaminierte Überdeckungsbeton abgetragen, der Bewehrungsstahl gereinigt und mit einem neuen Korrosionsschutz versehen (z. B. auf Polymer- oder Zementbasis). Der instandgesetzte Bereich hält häufig jedoch nur wenige Jahre (aufgrund mechanischer, thermischer und/oder hygrischer Inkompatibilitäten), so dass eine zeitnahe weitere Instandsetzung erforderlich wird, gerade dann, wenn der Überdeckungsbeton stark beansprucht wird.

Insgesamt besteht somit aus Sicherheitsaspekten der Wunsch und die Notwendigkeit den Zustand des Stahlbetonbauwerkes im Hinblick auf viele verschiedene Systemparameter regelmäßig zu überwachen, um Wartungs- oder Instandsetzungsintervalle zu kennen und rechtzeitig das Stahlbetonbauwerk zu sanieren. Bislang wurden bei den wenigstens Bauwerken betontechnologische Daten erfasst und verarbeitet oder aber nur einzelne Systemparameter unabhängig voneinander durch im Bauwerk eingebaute Sensorsysteme überwacht. Dabei fehlte es an der Berücksichtigung von kombinierten chemischen, mechanischen, physikalischen und meteorologischen Einflüssen auf das Bauwerk. Diese unabhängige Betrachtung führte bislang dazu, dass die verwendeten Sensorsysteme selbst gewartet werden mussten und für jeden eine eigene Stromversorgung notwendig war, da ein Batteriebetrieb selten eine ausreichende Messdauer garantieren konnte.

Die Aufgabe der vorliegenden Erfindung ist es daher ein Verfahren zur Überwachung eines Bauwerkes aus Stahlbeton anzugeben, welches besonders einfach, umfassend und energieeffizient den Zustand eines Bauwerkes erfasst.

Die Aufgabe wird erfindungsgemäß gelöst, indem die Ansteuerung der Sensorelemente und/oder die Auswertung der gemessenen Sensordaten durch die Sensorelemente durch die Cloud oder die externe Auswerteeinheit initiiert bzw. durchgeführt wird.

Die Erfindung geht dabei von der Überlegung aus, dass für eine besonders gute und umfassende Zustandsanalyse des Stahlbauwerks alle möglichen und insbesondere relevanten Daten von einer zentralen Cloud oder externen Auswerteeinheit verarbeitet werden. Insbesondere dann, wenn bereits eine Grundanalyse der durch die Sensorelemente gemessenen Daten des Bauwerks durch die Cloud oder externe Auswerteeinheit durchgeführt wurde, können gezielt einzelne Sensorelemente angesteuert werden, wenn beispielsweise in diesem örtlichen oder in diesem messtechnischen Bereich, insbesondere einem einzelnen Messparameter, eine Nachmessung erfolgen soll. Aus diesem Grund ist die Cloud oder die externe Auswerteeinheit dazu ausgebildet einzelne Sensorelemente anzusteuern und eine Messung zu initiieren.

Das System selbst umfasst ein im Wesentlichen drahtloses Sensornetz, um die Veränderungen der physikalischen und chemischen Eigenschaften der Strukturen zu erfassen und an die Server in der Cloud oder zu externen Auswerteeinheiten zu übertragen. Die Daten werden verarbeitet und für die Überwachung und Diagnose des aktuellen Zustands der Strukturen sowie für die Vorhersage der verbleibenden Lebensdauer der Struktur verwendet. Dabei ist es insbesondere möglich über numerische Simulationen, Verwendung von Algorithmen aus dem Bereich der künstlichen Intelligenz, Erfahrungswerten aus anderen Gebäuden und vergangenen Simulationen, gerade im Bereich der Migrationsprozesse von Chloriden oder CO₂ in Abhängigkeit der Betonart und -güte, Feuchtigkeit und Temperaturen eine umfassende Bewertung des Bauwerkszustandes zu ermitteln und entsprechende Wartungs-, Instandhaltungs- oder Reparaturzyklen zu bestimmen. Die im Rahmen der Messungen erhaltenen Daten können dabei insbesondere in einem umfassenden 3D-Modell dargestellt werden, wodurch es den Benutzern ermöglicht wird einen vollständigen Überblick über den Bauwerkzustand zu erhalten. Zu den Bauwerken, bei denen das System eingesetzt werden kann, gehören Brücken, Tunnel, mehrstöckige Gebäude und Parkhäuser, Windkraftanlagen und Staudämme.

Das System ist so konzipiert, dass es die Komplexität der Sensorknoten reduziert, indem es die Verarbeitungsprozesse in die Cloud verlagert. Dies führt zu einer kostengünstigen Lösung, die für den Einsatz in mehr Strukturen geeignet ist.

Da die einzelnen Endgeräte bei Bedarf durch die Cloud bzw. die dort befindlichen Server oder die externe Auswerteeinheit angesteuert werden können, können diese bevorzugt deaktiviert werden, wenn diese gerade nicht gebraucht werden. Mit deaktiviert wird dabei eine Art Ruhezustand verstanden, in denen alle Sensorelemente inaktiv sind und auch im Endgerät selbst alle wesentlichen, stromverbrauchenden Elemente ausgeschaltet werden. Lediglich eine Ansteuereinheit zur Aktivierung durch die Cloud bzw. die externe Auswerteeinheit bleibt aktiv, um entsprechende Steuerbefehle empfangen zu können. Dadurch ist es möglich die Endgeräte oder Sensoren fest im Stahlbetonbauwerk zu verbauen und ohne Stromanschluss und nur mit Batteriebetrieb zu betreiben. Durch den Ruhemodus sind dabei Batterielaufzeiten von bis zu 15 Jahre möglich. Dies senkt die Wartungskosten des Systems, insbesondere bei Bauwerken, bei denen der Zugang zu den Sensoren schwierig ist, wie z. B. an der Spitze von Masten und Säulen oder bei Sensoren, die in das Baumaterial eingebettet sind.

Im Gegensatz zu den Endgeräten, sind Router Kommunikationsknoten mit einem eigenen Stromversorgungsanschluss, bei denen der Transceiver stets aktiviert ist. In einer bevorzugten Ausgestaltung sind daher Router vorgesehen, die zwischen den Endgeräten und dem Gateway angeordnet sind und die Kommunikationspakete weiterleiten. Um das gesamte Bauwerk zu vernetzen, sind in vorteilhafter Ausführung Routerketten vorgesehen, wobei jeder Router mit einer Anzahl an Endgeräten kommuniziert und mit weiteren Routern, beispielsweise mit den beiden in der Kette benachbarten Routern. In einer bevorzugten Ausführungsform kann ein Endgerät, welches an einer permanenten Stromversorgung angeschlossen ist, ebenfalls als Router fungieren. In diesem Fall geht das Endgerät selbst nicht einen Ruhezustand, sondern bleibt stets aktiv und übernimmt zusätzlich die Funktion eines Routers.

Wie bereits erwähnt werden die Endgeräte bevorzugt deaktiviert bzw. in einen Ruhezustand versetzt, solange sie gerade nicht gebraucht werden bzw. sofern gerade keine Messung durch eines ihrer Sensorelemente benötigt wird. In einer bevorzugten Ausgestaltung der Erfindung ist daher ein Messzeitplan im System hinterlegt nach dem die einzelnen Sensorelemente angesteuert und aktiviert werden, um eine Messung zu initialisieren. Dieser Messzeitplan kann dabei einmalig hinterlegt werden oder aber auch in vorteilhafter Ausführung je nach Zustand des Bauwerkes und der bisherigen Messwerte aktualisiert werden, sodass gewisse Sensorelemente, in denen kritische Messwerte erwartet werden, häufiger als ursprünglich geplant überwacht und gemessen werden können. Auch hier kommen die oben beschriebenen numerischen Simulationen, Verwendung von Algorithmen aus dem Bereich der künstlichen Intelligenz, Erfahrungswerte aus anderen Gebäuden und vergangenen Simulationen bei der Beurteilung der Sensorwerte zum Einsatz.

Die Darstellung der Ergebnisse kann dabei auf mehrere Arten erfolgen. So ist es möglich die Rohdaten und daraus abgeleitete Parameter darzustellen, ebenso ist eine graphische Darstellung der Messwerte in verschiedenen Diagrammen mit eingezeichneten Grenzwerten (insbesondere auch Vorgrenzwerte, Grenzwerte und kritische Werte) möglich. Ebenso ist die Darstellung in Form einer Gebäudeampel möglich, in der alle oder zumindest die meisten Messwerte und daraus abgeleiteten Parameter in einen Zustandsparameter zusammengefasst werden, der nur angibt, ob sich das Gebäude in einem unbedenklichen Zustand (beispielsweise mit der Farbmarkierung grün) in einem Zustand der benötigten Wartung (beispielsweise mit der Farbmarkierung gelb) oder in einem kritischen Zustand (beispielsweise mit der Farbmarkierung rot) befindet.

Ebenso ist es möglich einen einzelnen Messwunsch zu erzeugen, das bedeutet, dass einzelne oder eine Gruppe von Sensorelementen gezielt und außerhalb des Messzeitplanes aktiviert werden und eine Messung initiiert wird. Ein derartiger Messwunsch kann beispielsweise mit dem Ziel erzeugt werden, um ein einzelnes Messergebnis zu verifizieren oder aber wenn auf Basis anderer Messwerte ein kritischer Bauwerkszustand möglich scheint, diesen zu lokalisieren bzw. zu konkretisieren.

Für eine besonders einfache Verteilung von neuen Messparametern, wie beispielsweise Messzeitpläne, Messdauern, Messprotokolle oder Kommunikationsupdates, wie beispielsweise Kommunikationsprotokolle, werden auch diese über die Cloud bzw. die externe Auswerteeinheit zentral gesteuert und an die Endgeräte, Sensoren, Router und/oder das Gateway verteilt. Eine Besonderheit des Systems besteht darin, dass die Sensoren mit Hilfe von Netzwerksynchronisationstechniken oder GPS-Signalen im Bereich von Mikrosekunden oder sogar Nanosekunden synchronisiert werden können. Dadurch kann das System das Verhalten des Bauwerks als Ganzes in Reaktion auf die physikalische Umgebung, z. B. durch Verkehr, Wind oder Stöße verursachte Vibrationen, besser analysieren.

Je nach Bauwerk werden bevorzugt eine Vielzahl von verschiedenen Sensoren und insbesondere auch Sensortypen verwendet und über das Bauwerk verteilt, um einen ausreichenden Gesamtüberblick über den Zustand des Bauwerkes zu erhalten. Dabei ist es insbesondere von Vorteil eine Mischung aus mechanischen Sensoren und chemischen, insbesondere betontechnologischen Sensoren zu kombinieren.

Die Sensorelemente werden bevorzugt aus der Gruppe der Beschleunigungsmesser, GNSS-Sensoren, Dehnungsmessstreifen, Betonzustandssensoren, wie beispielsweise Anodenleiter, Sensoren zur Messung des elektrischen Widerstands, Karbonatisierungssensor, Referenzelektrode und Riss- und Fugensensoren ausgewählt.

Ein Beschleunigungsmesser, insbesondere ein 3D-Beschleunigungssensor wird zur Überwachung der dynamischen Eigenschaften der Strukturen verwendet. Beschleunigungssensoren erfassen Größe und Richtung der Bewegung im 3D-Raum. Diese Informationen können zur Überwachung der Veränderungen der dynamischen Eigenschaften der Struktur verwendet werden. Die dynamischen Eigenschaften der Struktur können sich aufgrund von Schäden oder Leistungsänderungen der Struktur ändern. Der Beschleunigungssensorknoten misst die Beschleunigung und überträgt die Rohdaten in bestimmten Intervallen, Zeiträumen und Raten zur Verarbeitung an die Cloud.

Ein GNSS-Sensorknoten verwendet einen hochpräzisen Global Positioning System-Empfänger, um seinen Standort mit einer Genauigkeit von weniger als einem Zentimeter zu messen. Mit diesem Gerät können Verformungen großer Strukturen erkannt und gemessen werden, die wiederum zur Korrektur des Strukturmodells verwendet werden können, wenn die dynamischen Eigenschaften der Struktur z. B. anhand von Beschleunigungsmessdaten bewertet werden.

Ein Dehnungsmessstreifen-Sensorknoten wird zur Messung von Dehnungen in verschiedenen Bauteilen wie Stahl oder Stahlbeton verwendet. Er kann Dehnungen in Pfählen, Fundamenten, Dämmen, Tunneln usw. überwachen. Die Hauptkomponente des Vibrationssensors ist ein gespannter Stahldraht, der beim Ziehen mit einer Resonanzfrequenz schwingt, die proportional zur Dehnung im Draht ist. Dieser Mechanismus wird zur Messung von statischer Dehnung, Spannung, Druck, Neigung und Verschiebung durch verschiedene Sensorkonfigurationen verwendet.

Der Betonzustandssensorknoten ist eine Schnittstelle zu vielen verschiedenen Sensoren, die jeweils eine andere Eigenschaft des Betons oder seiner Umgebung messen, um den aktuellen Zustand der Korrosion der Stahlbewehrung im Beton zu überwachen. Zusätzlich zu den Sensoren für die Betonfeuchte und -temperatur, die Temperatur der Anodenleiter, Multiringelektroden, Karbonatisierungssensoren, Leitfähigkeitssensoren, die Luftfeuchtigkeit und die Lufttemperatur können ebenfalls Anodenleiter, Sensoren für den elektrischen Widerstand, Referenzelektroden und Riss- und Fugensensoren an den Betonzustandssensorkonten angeschlossen. Derselbe Knoten kann auch für die Überwachung der Korrosion anderer Bauwerke wie z. B. Eisenbahnstrecken verwendet werden.

Ein Anodenleiter umfasst vier oder mehr Anoden mit gleichem Abstand zueinander, die in den Beton eingebettet sind. Der Sensorknoten misst den elektrischen Wechselstromwiderstand zwischen den Anoden und berechnet einen Parameter namens linearer Polarisationswiderstand des Betons, der ein Indikator für die Korrosion des Bewehrungsstahls im Beton ist. Eine Zulagekathode ermöglicht die Messung des Korrosionsstroms. Alternativ ist die Messung des Korrosionsstromes aber auch über den linearer Polarisationswiderstand möglich.

Multiringelektroden werden zur Bestimmung der Feuchteverteilung in der Betonrandzone bei wasserbeaufschlagten Bauteilen und zur Überwachung der Wirksamkeit von Abdichtungen und Beschichtungssystemen z. B. unter Brückenkappen oder in Fugenbereichen eingesetzt. Dabei ist es möglich Multiringelektroden direkt als auch nachträglich einzubauen.

Karbonatisierungssensoren messen den elektrischen Widerstand in verschiedenen Tiefen des Betons in etwa 4 mm dicken Schichten, um die Karbonatisierungstiefe zu bestimmen. Um den Widerstand gegen die Karbonatisierung besser bestimmen zu können werden zusätzlich die Temperatur und die relative Luftfeuchtigkeit in einem kleinen Hohlraum im Beton gemessen und für die Auswertung herangezogen.

Ein Sensor des elektrischen Widerstandes wird dabei zur Überwachung der Korrosionsrate und des Ausmaßes des Gesamtmetallverlustes bei allen metallischen Ausrüstungen oder Strukturen verwendet. Die Sonde selbst besteht aus einem Metallelement, das der Umgebung ausgesetzt ist. Da die Korrosion stattfindet, führt der Metallverlust zu einer Verringerung des Querschnitts des Elements aufgrund der Korrosion, was mit einem proportionalen Anstieg des elektrischen Widerstands des Elements einhergeht. Der elektrische Widerstand wird vom Sensorknoten gemessen, um den Korrosionsfortschritt anzuzeigen.

Ein Riss- bzw. Fugensensorknoten misst die Verschiebung über Risse und Fugen eines Bauwerks. Wenn sich der Riss ausdehnt oder zusammenzieht, bewirkt die Änderung des Abstands zwischen den Ankern eine Bewegung der Verbindungsstange in einem Potentiometerkörper. Über die Bewegung des Potentiometers kann die Bewegung des Risses mit hoher Auflösung und Genauigkeit bestimmt werden. Alternativ können auch faseroptische Risssensoren zur Anwendung kommen.

Bei den Referenzelektroden war es bislang üblich Referenzelektroden mit Festkörpermembranen für den Einsatz im Beton einzusetzen. Diese Referenzelektroden (Bezugselektrode) dienen zur Messung von relativen Potentialen anderer Elektroden mit konstantem Gleichgewichtspotential. Sie bestehen allgemein aus einer Elektrode und einem Elektrolyt, wobei das System mittels einer Membran von seiner Umgebung getrennt wird. Die Membran ist dabei derart ausgebildet, dass Ionen durch sie diffundieren können. Diese Referenzelektroden werden dauerhaft in den Beton eingebracht. Entweder bereits am Anfang beim Aufbau eines kathodischen Korrosionsschutzes oder aber nachträglich durch Bohrungen in den Beton, um eine elektrische Verbindung mit dem Beton zu ermöglichen. In beiden Fällen werden die Referenzelektronen fest im Beton verbaut und können nicht flexibel für mehrere Bauwerke oder Positionen verwendet werden. Eine wirksame Überwachung des gesamten Bauwerkes ist dabei nur möglich, wenn ausreichende viele Referenzelektroden im Beton eingesetzt und über das Bauwerk verteilt werden, was die Kosten für eine solche Anlage deutlich erhöht.

Neben der Chloridkorrosion von Stahlbetonbauwerken, die durch den kathodischen Korrosionsschutz überwacht, kontrolliert und auf ein vernachlässigbares Maß reduziert werden kann, kann mittels Referenzelektroden aber auch die Korrosion durch Karbonatisierung von Stahlbetonbauwerken überwacht werden.

Weiterhin ist die Verwendung von Elektroden bekannt, die als Kontaktelement einen feuchten, elektrolytgetränkten Schwamm aufweisen, um die Elektrode und den Beton miteinander elektrisch zu verbinden. Nachteilig hierbei ist allerdings, dass der Elektrolyt zu schnell aus dem Schwamm bzw. der Referenzelektrode austritt, weshalb die Referenzelektrode schnell austrocknet und ersetzt werden muss. Darüber hinaus wird durch die austretende hoch leitfähige Elektrolytlösung bei dieser Variante das Messergebnis durch die Durchfeuchtung des Umgebungsbetons instabil und daher nur für die Kurzzeitmessung an exemplarischen Bauteilen geeignet.

In vorteilhafter Ausführung wird hier allerdings eine Referenzelektrode verwendet, bei der das Kontaktelement als elastischer Feststoff ausgebildet ist. Die Überlegung dahinter ist, dass ein flexibler Einsatz der Referenzelektrode insbesondere dann erreicht werden kann, wenn auf den festen Einbau einer Referenzelektrode durch Einmörteln in das Bauwerk verzichtet werden kann. Für eine zuverlässige Messung des Potentials wurde aber festgestellt, dass eine ausreichend hohe Kontaktfläche der Referenzelektrode mit dem Beton des Bauwerkes vorliegen sollte. Da die bisherigen Ausführungen mit Festkörpermembranen dazu nicht geeignet waren, wurde ein Kontaktelement entwickelt, welches die Festkörpermembran ersetzt. Dieses Kontaktelement wurde dabei gezielt elastisch ausgebildet. Bevorzugt ist das Kontaktelement zumindest wasserhemmend, eher wasserabweisend oder sogar wasserundurchlässig ausgebildet. Dies bedeutet, dass das Kontaktelement bevorzugt einen Wasseraufnahmekoeffizient von w ≤ 2 kg/(m²·h^{0,5}) aufweist. Der Wasseraufnahmekoeffizient (kurz w-Wert) gibt an, wie viel Wasser ein Stoff innerhalb einer bestimmten Zeit aufnimmt bzw. aufnehmen kann. Dabei werden Baustoffe mit einem w-Wert von unter 2 kg/(m²·h^{0,5}) als wasserhemmend bezeichnet. Bevorzugt ist das Material des Kontaktelements sogar wasserabweisend (w ≤ 0,5 kg/(m2·h0,5)) oder besonders bevorzugt sogar wasserundurchlässig (*w* ≤ 0,001 kg/(m²·h^{0,5})). Durch diese Elastizität fügt sich das Kontaktelements beim Aufdrücken der Referenzelektrode auf den Beton des Bauwerkes an die körnige Oberfläche des Betons an, dringt zumindest teilweise in die Poren ein und erzeugt eine besonders gute und große Kontaktfläche zwischen der Referenzelektrode und dem Beton, ohne dass der Elektrolyt aus der Referenzelektrode austritt und in die Betonumgebung eindringt. Bei einer derartigen Referenzelektrode sorgt das Kontaktelement einerseits für eine besonders große Kontaktfläche mit dem Beton und fungiert andererseits als sogenannte Salzbrücke. Das bedeutet, es gibt keinen direkten Kontakt zwischen Elektrode und Beton, womit eine Entladung im Elektrolyt zeitlich herausgezögert bzw. teilweise vermieden werden kann.

Um einerseits eine besonders große Kontaktfläche des Kontaktelements auf dem Beton beim Anpressen der Referenzelektrode zu erhalten und andererseits aber auch zu verhindern, dass das Kontaktelement aufgrund des Anpressdruckes auf dem Beton bricht, wird bevorzugt ein Material für das Kontaktelement verwendet, welches eine elastische Dehnung im Bereich von 5 % bis 50 % aufweist, besonders bevorzugt im Bereich von 15 % bis 35 %.

Für die Potentialmessung ist das Kontaktelement ionisch leitfähig ausgebildet. Dabei ist Dicke des Kontaktelements aufgrund der gewünschten Leitfähigkeit bevorzugt klein gehalten. In vorteilhafter Ausgestaltung ist die Dicke zwischen 1 mm und 20 mm, bevorzugt im Bereich von 5 mm bis 15 mm. Die Wahl der Dicke des Kontaktelements kann dabei an die Rauigkeit des Betons und an die Elastizität des Kontaktelements angepasst werden, um so einen optimalen Mittelweg zwischen möglichst großer Leitfähigkeit und möglichst großer Kontaktfläche zu finden.

In bevorzugter Weise ist das Kontaktelement einerseits dazu ausgebildet die Kammer mit dem Elektrolyt dicht zu verschließen, andererseits elastisch genug, um eine große Kontaktfläche auf dem Beton bereitzustellen und weiterhin möglichst leitfähig, um eine zuverlässige Potentialmessung zu ermöglichen. Vorteilhaft ist das Kontaktelement daher aus Silikon oder einem Elastomer mit freien Ionen gebildet. Bei der Verwendung von elastischen Materialien, wie beispielsweise Polyurethanen oder Silikonkautschuk, die für sich ursprünglich keine ausreichend hohe Leitfähigkeit aufweisen, kann durch Zugabe von leitfähigen Stoffen, beispielsweise einer leitfähigen Flüssigkeit wie Ca(NO₃)₂, die gewünschte Leitfähigkeit hergestellt werden. Alternativ können ebenfalls elastische Materialien verwendet werden, die mit ionisch funktionalen Gruppen ausgebildet sind, wie beispielsweise ionische Polymere, zu denen beispielsweise DADMAOH gehört. Als Elektrolyt bevorzugt Kupfersulfat verwendet.

Eine derartige Referenzelektrode wird bevorzugt beim Betreiben eines kathodischen Korrosionsschutzes und/oder bei der Überwachung von Korrosionszuständen eingesetzt. Dabei muss die Referenzelektrode nicht wie bislang notwendig in den Stahlbeton dauerhaft eingebracht werden, sondern kann diese in einem Bohrloch oder sogar auf der Betonoberfläche anpressen, um die Potentiale bestimmen zu können. Aus dieser Potentialmessung lässt sich der Zustand der Bewehrung überwachen und im Falle des kathodischen Korrosionsschutzes entsprechende Regelparameter für Strom und Spannung ermitteln. Bei Bedarf ist aber auch hier ein Einbau durch Einmörteln möglich.

Vorteile bei der Verwendung einer solchen Referenzelektrode sind insbesondere, dass durch die Verwendung eines elastischen Kontaktelements eine besonders einfache und gute Kontaktierung der Referenzelektrode und somit Potentialmessung erreicht werden kann. Dadurch müssen die Referenzelektronen bei Installation der Anlage oder nachträglich nicht fest verbaut werden, sondern können mehrfach und flexibel verwendet werden. Auch wird durch das verwendete Kontaktelement ein Austritt der Elektrolytlösung aus der Referenzelektrode verhindert, wodurch eine Belastung des Betons am Messpunkt vermieden wird und zukünftige Messungen aufgrund der ausgetretenen Elektrolytlösung und somit einer anderen Materialzusammensetzung am Messpunkt nicht verfälscht werden.

Durch die Verwendung der oben beschriebenen Referenzelektrode ist es nun ebenfalls möglich Stahlbetonbauwerke an besonders korrosionsgefährdeten Bereichen, mit einer Vielzahl an Referenzelektroden dauerhaft zu überwachen. Dies kann sowohl bei Neubauten, die zum Beispiel aus Kostengründen ohne Beschichtung gebaut werden, als auch bei Bauwerken, die bereits deutliche Korrosionserscheinungen aufweisen, eingesetzt werden, um den richtigen Zeitpunkt einer Sanierungsmaßnahme festlegen zu können. Bislang mussten solche Bestandsbauten teuer untersucht werden, da in der Regel ohne das Entfernen der vorhandenen Beschichtungen keine Potenziale gemessen werden konnten.

Vereinzelt ist es trotzdem möglich oder vorteilhaft, dass trotz der hier beschriebenen Verfahren zur energieeffizienten Vernetzung der Endgeräte, welches in vielen Bereichen die Möglichkeit bietet auf teure und schwer zu installierende externe Stromversorgungen zu verzichten, einzelne Endgeräte bzw. Sensorelemente des Überwachungssystems an eine externe Stromversorgung angeschlossen werden. Dies ist insbesondere für die Endgeräte denkbar, die im Dauerbetrieb arbeiten, wie beispielsweise ein 3D-Anemometer oder Verkehrskameras zur Überwachung des Fahrzeugverkehrs, um die Umweltauswirkungen und Belastungen auf das Gebäude (wie beispielsweise eine Brücke) zu überwachen. Derartige Sensorknoten, die so viel Energie verbrauchen, dass ein Batteriebetrieb nicht möglich oder praktikabel ist, können dabei auf zwei verschiedene Arten im Überwachungssystem integriert werden. Einerseits kann dies über eine geeignete Hardwareschnittstelle, die speziell für die Verbindung mit diesem spezifischen Sensor entwickelt wurde realisiert werden. Diese Hardwareschnittstelle ist dabei dazu ausgelegt die Daten vom Sensor zu lesen und in das Protokoll und Format des Überwachungssystems zu wandeln und in das Überwachungssystem einzuspeisen. Andererseits ist aber auch eine Softwareschnittstelle denkbar, die die Daten aus der lokalen oder cloudbasierten Software des Sensors sammelt, sie in das Protokoll und Format des Überwachungssystems übersetzt und über eine Internetverbindung oder direkt in der Cloud an das Überwachungssystem weiterleitet.

Die mit der Erfindung erzielten Vorteile bestehen insbesondere darin, dass durch die zentrale Auswertung und Steuerung des Überwachungssystems und die Verwendung eines Mesh-Netzwerkes mit einer Vielzahl von Endgeräten, diese gezielt und schnell angesteuert werden können, um so einzelne Messungen zu initiieren. Gleichzeitig kann das System die einzelnen Endgeräte bzw. Sensorelemente gezielt in eine Art Ruhezustand versetzen, um Strom zu sparen. Dadurch ist ein Batteriebetrieb der Endgeräte und Sensorelemente mit einer sehr langen Lebensdauer und somit geringeren Wartungsintervallen möglich. Auch können Updates von Messparametern oder Kommunikationsupdates leicht über die Cloud bzw. externe Auswerteeinheit auf dem System verteilt werden.

Ein Ausführungsbeispiel der Erfindung wird anhand einer Zeichnung näher erläutert. Darin zeigt:
- FIG. 1: ein Stahlbetonbauwerküberwachungssystem,
- FIG. 2: eine Routerkette,
- FIG. 3: ein Stahlbetonbauwerküberwachungssystem an einer Brücke,
- FIG. 4: ein Stahlbetonbauwerküberwachungssystem an Eisenbahnschienen,
- FIG. 5: ein Stahlbetonbauwerküberwachungssystem in einem Parkhaus,
- FIG. 6: ein Stahlbetonbauwerküberwachungssystem in einem Tunnel,
- FIG. 7a bis 7c: ein Karbonatisierungssensor,
- FIG. 8: eine Referenzelektrode mit einem elastischen Kontaktelement in Explosionsdarstellung,
- FIG. 9: ein System zur Messung des Korrosionspotentials an der Betonoberfläche mit einer Referenzelektrode,
- FIG. 10: ein System zur Messung des Korrosionspotentials mit einer Referenzelektrode in einem Bohrloch,
- FIG. 11: ein System zur Messung des Korrosionspotentials mit einer Referenzelektrode mit einem Gewinde in einem Bohrloch.

Gleiche Teile sind in allen Figuren mit denselben Bezugszeichen versehen.

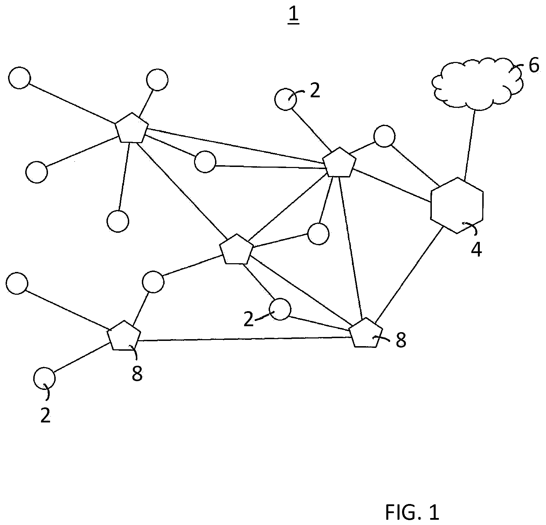

Ein Ausführungsbeispiel eines Stahlbetonüberwachungssystems 1 ist in FIG. 1 dargestellt. Dieses Stahlbetonüberwachungssystem besteht aus einer Anzahl von Endgeräten 2, die entweder einen oder mehrere Sensoren beinhalten und/oder mit einem oder mehreren Sensoren gekoppelt sind, sodass diese Sensoren durch das Endgerät 2 aktiviert und/oder angesteuert werden können und Daten vom Sensor durch das Endgerät 2 empfangen, bearbeitet und weitergeleitet werden können. Das Stahlbetonüberwachungssystem 1 umfasst darüber hinaus ein Gateway 4, welches die Kommunikationsschnittstelle zwischen den Endgeräten 2 und einer Cloud 6 bildet. Die Cloud 6 ist einerseits dazu ausgebildet, die Daten der Endgeräte 2 bzw. der Sensoren zu sammeln, auszuwerten und somit eine Aussage über den Zustand des Bauwerkes zu treffen, um gegebenenfalls auch Warn- oder Wartungshinweise zu erzeugen. Umgekehrt ist die Cloud 6 aber auch dazu ausgelegt die Endgeräte 2 und gegebenenfalls die Sensoren zu konfigurieren und bei Bedarf gezielte Sensormessungen zu initiieren, um beispielsweise nicht eindeutig ermittelbare Zustandsgrößen des Bauwerkes zu verifizieren.

Das Stahlbetonüberwachungssystem 1 nach der FIG. 1 umfasst darüber hinaus eine Anzahl von Routern 8, die zwischen den Endgeräten 2 und dem Gateway 4 geschaltet sind. Wie in der FIG. 1 dargestellt können einzelne Endgeräte 2 aber auch direkt signaltechnisch an das Gateway 4 angeschlossen werden. Die Router 8 dienen dabei insbesondere als Kommunikationsknoten mit einem eigenen Stromversorgungsanschluss, bei denen der Transceiver stets aktiviert ist. Dies ermöglicht es die Endgeräte 2 örtlich nah an den Sensoren anzuordnen, sofern die Endgeräte 2 nicht sogar die Sensoren beinhalten, und hauptsächlich in eine Art Ruhemodus zu betreiben. Die Endgeräte 2 werden daher im Wesentlich nur im Rahmen der Messung und Weiterleitung der Sensordaten und im Rahmen von neuen Konfigurationsbefehlen durch die Cloud aus dem Ruhemodus geholt. Dadurch können die Endgeräte 2 sehr stromsparend und insbesondere mit Batterien betrieben werden. Auf eine aufwändige Stromverkabelung an alle Endgeräte 2 und Sensoren kann somit weitestgehend verzichtet werden.

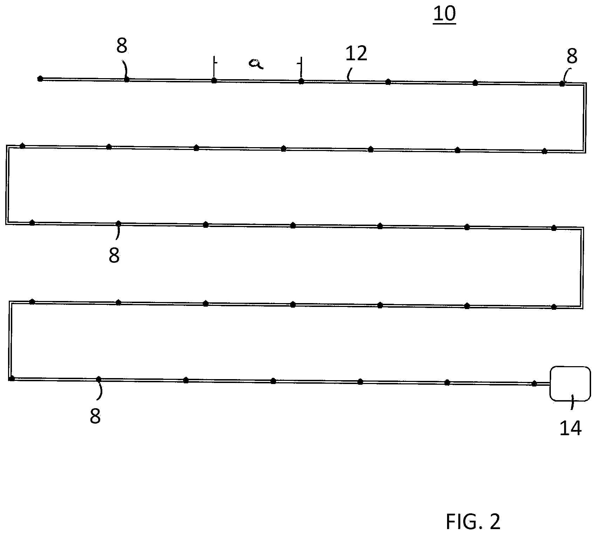

Die Router 8 sollten sich über die gesamte Fläche eines Bauwerkes erstrecken. Außerdem müssen sie an eine konstante Stromquelle angeschlossen werden, in diesem Fall eine Niederspannung wie 12 oder 24 Volt. Um die Installation einfach und schnell umsetzen zu können, ohne dass jeder Router eine eigene Stromversorgung aufweisen muss, werden Router als Schnur auf einem Draht hergestellt und auf einer Rolle geliefert. Eine derartige Routerkette 10 ist in FIG. 2 beispielhaft dargestellt. Für die Verbindung der Router 8 wird ein zweiadriges Kabel 12 mit geringem Widerstand verwendet und die Router 8, die sehr klein sind, werden etwa alle Abstand a etwa alle 15 Meter entlang des Kabels 12 angeschlossen. Diese Strings können in Längen von Hunderten von Metern hergestellt werden. Für noch längere Installationen, wie beispielsweise bei Brücken notwendig, können zwei Routerketten 10 an ihren Enden verbunden werden und Routerketten 10 von bis zu einem Kilometer gebildet werden. An einem Ende der Installation wird die Routerkette 10 dann an eine Stromversorgung 14 angeschlossen.

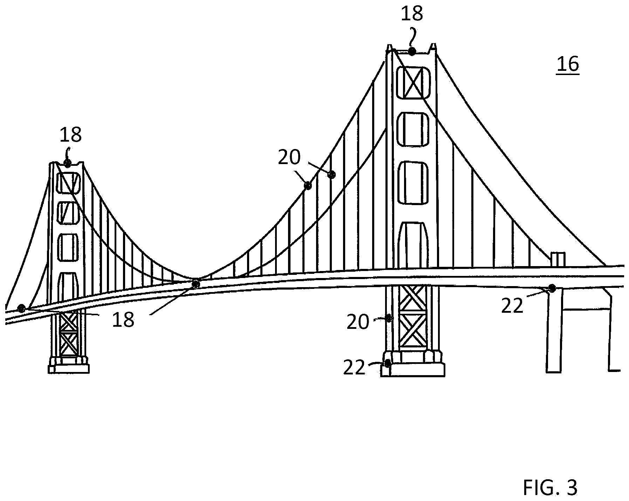

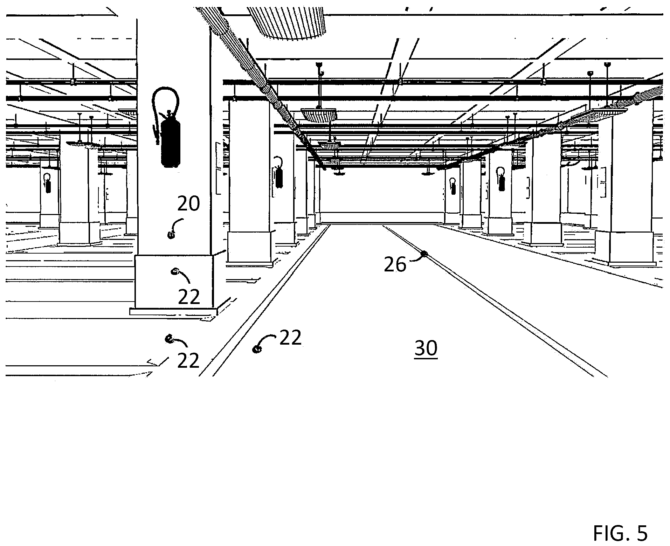

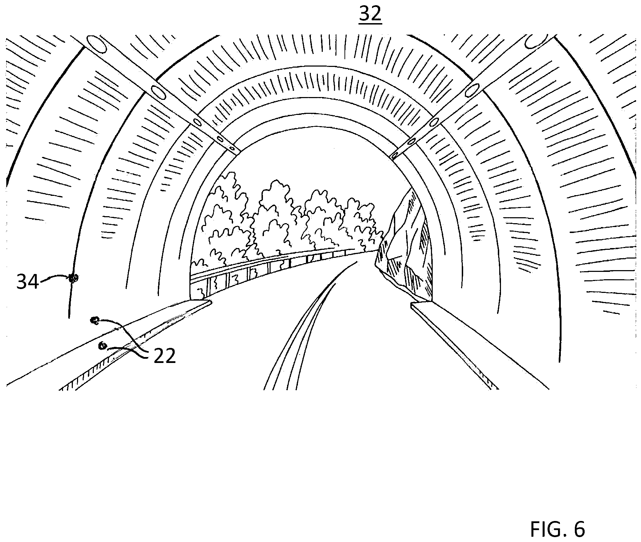

Beispielhafte Ausgestaltungen von Stahlbetonüberwachungssystemen 1 für verschiedene Bauwerke sind in den Figuren 3 bis 6 dargestellt. In all diesen Bauwerken ist ein System nach der FIG. 1 mit einem Gateway 4, einer Cloud 6, einer Vielzahl von Endgeräten 2 mit entsprechenden Sensoren und einem System aus Routern 8, beispielsweise in Form einer Routerkette 10 nach FIG. 2 verbaut.

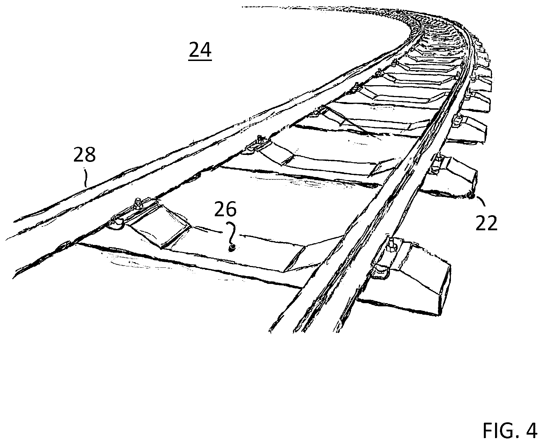

Die verwendeten Sensoren werden dabei in Abhängigkeit des zu überwachenden Bauwerkes und der typischen Einflussfaktoren auf die Bauwerke ausgewählt. Die Brücke 16 nach FIG. 3 umfasst dabei insbesondere eine Mehrzahl von GNSS-Sensoren 18 zur Bestimmung von Beschleunigungsmessdaten und eine Anzahl von Dehnungsmessstreifen 20 und Betonzustandssensoren 22. Die Eisenbahnschienen 24 nach FIG. 4 umfassen ebenfalls Betonzustandssensoren 22, aber auch Beschleunigungssensoren 26 und Sensoren zu Bestimmung der Korrosion 28. Der Stahlbeton in einem Parkhaus 30 kann insbesondere durch Betonzustandssensoren 22, Beschleunigungssensoren 26 und Dehnungsmessstreifen 20 überwacht werden. In einem Tunnel 32 werden insbesondere Betonzustandssensoren 22 und Riss- bzw. Fugensensoren 34 eingesetzt.

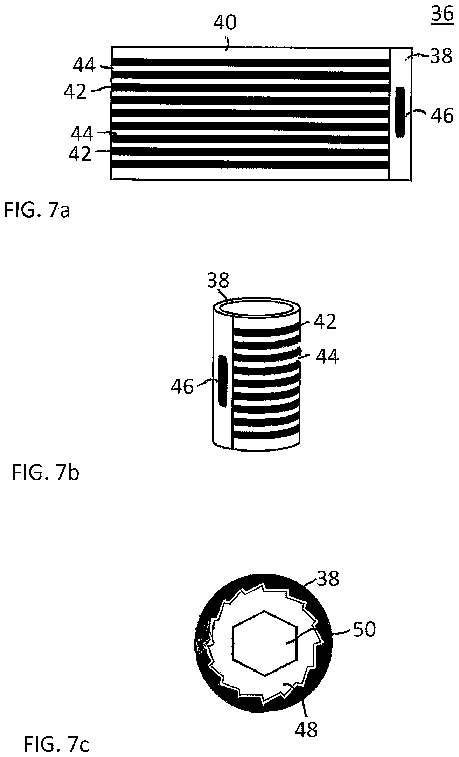

Ein Ausführungsbeispiel eines Karbonatisierungssensors 36 ist in den Figuren 7a - 7c dargestellt. Der Karbonatisierungssensor 36 umfasst dazu eine Elektrode 38, die aus einer flexiblen Leiterplatte 40 besteht, die im dargestellten Ausführungsbeispiel nach FIG. 7b und 7c zu einem Zylinderelement geformt ist. Auf dieser flexiblen Leiterplatte 40 sind eine Anzahl von Leiterbahnen 42 aufgedruckt, die im Abstand von etwa 3-4 mm ringförmig auf der Außenseite der zylinderförmigen Elektrode 38 angeordnet sind. Um die Oberfläche der Leiterbahnen 42 langfristig zu schützen, die Lebensdauer der Elektrode somit zu erhöhen, können die Leiterbahnen 42 mit Gold beschichtet werden. Zusätzlich werden die Leiterbahnen 42 auf der Leiterplatte 40 von einer Schicht aus leitfähigem, flexiblem Polymer bedeckt. Die Leiterbahnen 42 sind gegeneinander isoliert, dazu ist die Leiterplatte 40 zwischen den Leiterbahnen 42 mit einem nicht leitenden und ebenfalls flexiblen Isolationsmaterial 44 bedeckt. Dieses kann beispielsweise mit Hilfe der 3D-Drucktechnik auf die Leiterplatte 40 aufgebracht werden.

Aufgrund der Flexibilität der verwendeten Materialien, ist es möglich, dass die Elektrode 38 die Betonoberfläche möglichst großflächig bedeckt und somit einen gleichmäßigen und zuverlässigen Kontakt mit dem Beton herstellt.

Der Karbonatisierungssensor umfasst zusätzlich einen kleinen Messraum 46, der in Zylinderhöhe etwa mittig angeordnet ist. In diesem Messraum 46 können weitere Sensoren angeordnet werden. In bevorzugter Ausgestaltung sind zumindest ein Temperatur- und ein Feuchtigkeitssensor vorgesehen, die die Lufttemperatur und relative Luftfeuchte der Luft in dem Hohlraum des Messraumes 46 messen. Mit Hilfe dieser beiden zusätzlichen Messwerte 46 kann die Karbonatisierung besser bestimmt werden.

Für den Einbau wird der Karbonatisierungssensor in ein vorgereinigtes Bohrloch im Beton eingesetzt und mit einem mechanischen Verriegelungsmechanismus 48 an die Betonwandungen des Bohrloches gepresst. Das flexible Material schmiegt sich dabei optimal an die Konturen der Betonwand an, damit ein optimaler Kontakt zwischen der Elektrode und der Betonwand hergestellt wird. In dieser Position wird der Hohlraum des Sensors mit Silikon oder Epoxidharz 50 gefüllt, um den Sensor vollständig abzudichten und in der Position zu halten.

Die Messung erfolgt, in dem an die obere und untere Leiterbahn 42 ein Potential angelegt wird und der Strom, der durch den Beton fließt, gemessen wird. Anschließend werden die Potentiale an allen anderen Leiterbahnen 42 gemessen und der Widerstand zwischen den Leiterbahnen anhand der gemessenen Werte berechnet. Zusammen mit den Umgebungswerten, insbesondere der Temperatur und relativen Luftfeuchte, kann daraus die Karbonatisierung bestimmt werden.

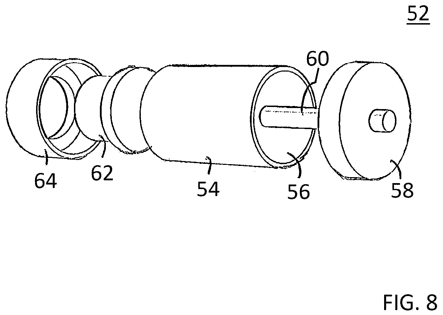

Die Referenzelektrode 52 nach FIG. 8 umfasst einen im Wesentlichen zylindrischen Grundkörper 54, der eine Kammer 56 bildet, die zumindest teilweise mit einem Elektrolyt gefüllt ist. Die eine Seite des Grundkörpers 54 wird mit einer Verschlusskappe 58 verschlossen, die eine Einführöffnung für eine Elektrode 60 aufweist, derart, dass die Elektrode 60 wenn die Verschlusskappe 58 auf dem Grundkörper 54 aufgesteckt oder aufgeschraubt ist in das Elektrolyt eintaucht. Weiterhin sind an der Verschlusskappe 58 oder direkt an der Elektrode 60 nicht dargestellte Anschlusseinrichtungen zur Verbindung elektrischer Kabel mit der Referenzelektrode 52 vorgesehen.

Auf der gegenüberliegenden Seite des Grundkörpers 54 ist ein Kontaktelement 62 vorgesehen, welches derart angeordnet ist, dass es einerseits im direkten Kontakt mit dem Elektrolyt in der Kammer 56 steht und andererseits eine genügend große Auflagefläche aufweist, die im Betrieb der Referenzelektrode 52 auf dem Beton aufliegt. Weiterhin ist eine Haltekappe 64 vorgesehen, die das Kontaktelement 62 in Position hält und die Kammer 56 verschließt, sodass das Elektrolyt nicht auslaufen kann. Das Kontaktelement 62 ist einerseits dazu ausgebildet die Kammer 56 mit dem Elektrolyt dicht zu verschließen, andererseits aber elastisch genug, um eine große Kontaktfläche auf dem Beton bereitzustellen und weiterhin möglichst leitfähig, um eine zuverlässige Potentialmessung zu ermöglichen.

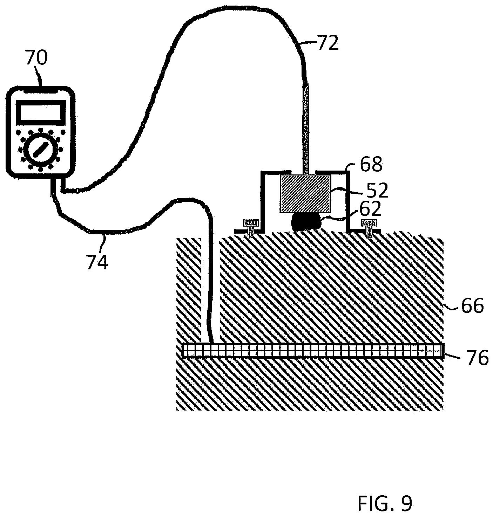

In FIG. 9 ist die Referenzelektrode 52 dargestellt. Diese Referenzelektrode 52 kann, muss aber nicht im Rahmen des kathodischen Korrosionsschutzes zum Einsatz kommen. Dabei wird die Referenzelektrode 52 auf den Beton 66 gedrückt und mittels Befestigungselementen 68 in dieser Position gehalten. Die Befestigungselemente 68 sind in FIG. 9 in Form von im Beton verschraubten Klammern dargestellt, es können aber auch andere Befestigungselemente 68 verwendet werden. Wie dargestellt formt sich das Kontaktelement 62 aufgrund seiner Elastizität besonders gut an die raue und unebene Betonoberfläche an, weshalb eine besonders große Kontaktfläche erreicht werden kann. Diese besonderes große Kontaktfläche bei gleichzeitig dichter Referenzelektrode ist ein besonderes Merkmal der Referenzelektrode 52 und sorgt für eine einfache, schnelle und dauerhafte Nutzung der Referenzelektroden 52.

Ein Spannungsmessgerät 70 ist über zwei Kabel 72, 74 einmal mit der Elektrode 58 und einmal mit der Stahlbewehrung 76 des Bauwerkes verbunden und kann über die Referenzelektrode 52 die angelegte Spannung messen. Dabei können über Spannungsänderungen im Vergleich zu früheren Messungen oder im Verlauf der Messung an der Referenzelektrode 52 auf den Zustand des Bewehrungsstahls und somit des Stahlbetons geschlossen werden, wodurch entweder das System zum kathodischen Korrosionsschutz eingestellt werden kann oder aber entsprechende Renovierungs- und Instandsetzungsmaßnahmen getroffen werden können.

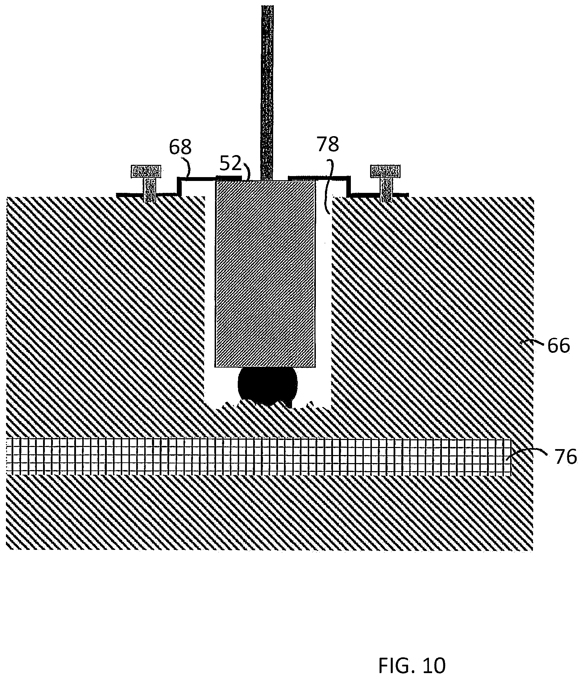

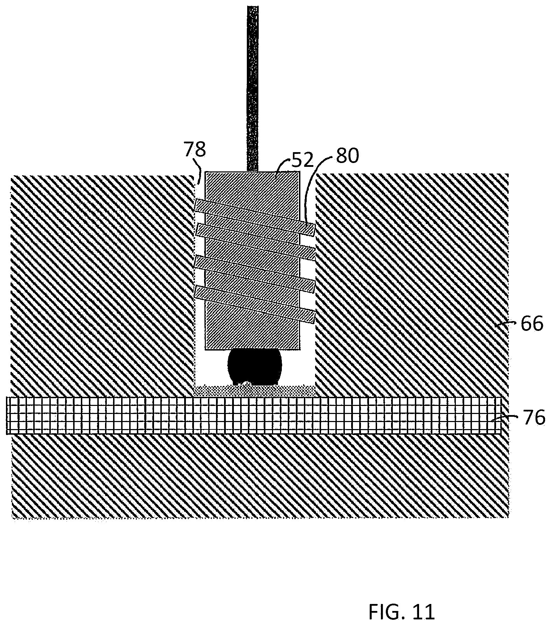

In alternativer Ausgestaltung kann die Referenzelektrode 52 im obigen System aber auch in anderen Anwendungen direkt in ein Bohrloch 78 des Betons 66 eingebracht werden. Dabei können die Befestigungselemente 68 wie in FIG. 10 dargestellt besonders flach ausgeführt werden, wodurch die Referenzelektrode 52 kaum sichtbar und geschützt vor Zugriffen bzw. äußeren Einflüssen ist. Alternativ kann, wie in FIG. 11 dargestellt, der Grundkörper 54 der Referenzelektrode 52 an der Außenseite ein Gewinde 80 aufweisen, wodurch die Referenzelektrode 52 direkt in das Bohrloch 78 eingeschraubt werden kann. Die übrigen Hohlräume des Bohrloches 78 können anschließend mit einem flüssigkeitsdichten Material ausgefüllt werden, damit sich im Bohrloch 78 kein Wasser sammelt, welches die Messung verfälschen könnte. Gerade der Einbau der Referenzelektrode 52 in ein Bohrloch 78 ist besonders vorteilhaft, da dadurch die Referenzelektrode besonders nah an der Stahlbewehrung 76 angeordnet werden kann.

### Bezugszeichenliste

- 1: Stahlbetonüberwachungssystem
- 2: Endgerät
- 4: Gateway
- 6: Cloud
- 8: Router
- 10: Routerkette
- 12: Kabel
- 14: Stromversorgung
- 16: Brücke
- 18: GNSS-Sensor
- 20: Dehnungsmessstreifen
- 22: Betonzustandssensoren
- 24: Eisenbahnschiene
- 26: Beschleunigungssensoren
- 28: Korrosionssensor
- 30: Parkhaus
- 32: Tunnel
- 34: Riss- bzw. Fugensensor
- 36: Karbonatisierungssensor
- 38: Elektrode
- 40: Leiterplatte
- 42: Leiterbahnen
- 44: Isolationsmaterial
- 46: Messraum
- 48: Verriegelungsmechanismus
- 50: Epoxidharz
- 52: Referenzelektrode
- 54: Grundkörper
- 56: Kammer
- 58: Verschlusskappe
- 60: Elektrode
- 62: Kontaktelement+
- 64: Haltekappe
- 66: Beton
- 68: Befestigungselement
- 70: Spannungsmessgerät
- 72: Kabel
- 74: Kabel
- 76: Stahlbewehrung
- 78: Bohrloch
- 80: Gewinde

## Patentansprüche

1. Verfahren zur Überwachung eines Bauwerkes aus Stahlbeton mit einer Mehrzahl von Endgeräten (2), wobei ein Endgerät (2) mindestens ein Sensorelement umfasst oder mit mindestens einem Sensorelement signaltechnisch verbunden ist und mindestens einem Gateway (4), wobei die Endgeräte (2) signaltechnisch direkt oder indirekt mit dem Gateway (4) verbunden sind und das Gateway (4) zur Kommunikation mit einer Cloud (6) oder einer externen Auswerteeinheit ausgebildet ist,
**dadurch gekennzeichnet, dass** die Ansteuerung der Sensorelemente und/oder die Auswertung der gemessenen Sensordaten durch die Sensorelemente durch die Cloud (6) oder die externe Auswerteeinheit initiiert bzw. durchgeführt wird.

2. Verfahren zur Überwachung eines Bauwerkes aus Stahlbeton nach Anspruch 1, **dadurch gekennzeichnet, dass** eine Anzahl von Routern (8) vorgesehen ist, die die Kommunikation zwischen Gateway (6) und aller oder einzelner Endgeräte (2) steuert.

3. Verfahren zur Überwachung eines Bauwerkes aus Stahlbeton nach Anspruch 1 oder 2, **dadurch gekennzeichnet, dass** einzelne oder alle Endgeräte (2) temporär deaktiviert werden können.

4. Verfahren zur Überwachung eines Bauwerkes aus Stahlbeton nach Anspruch 3, **dadurch gekennzeichnet, dass** die Endgeräte (2) auf Basis eines Messzeitplans aktiviert werden und einzelne oder alle angeschlossenen Sensorelemente eine Messung durchführen.

5. Verfahren zur Überwachung eines Bauwerkes aus Stahlbeton nach Anspruch 3 oder 4, **dadurch gekennzeichnet, dass** im Falle eines erkannten Messwunsches Steuersignale von der Cloud (6) oder der externen Auswerteeinheit an einzelne ausgewählte Endgeräte (2) geschickt werden, um die Endgeräte (2) zu aktivieren und eine Messung einzelner oder aller angeschlossener Sensorelemente dieser Endgeräte (2) zu initiieren.

6. Verfahren zur Überwachung eines Bauwerkes aus Stahlbeton nach Anspruch 5, **dadurch gekennzeichnet, dass** ein Messwunsch auf Basis der Auswertung anderer Messergebnisse erstellt wird.

7. Verfahren zur Überwachung eines Bauwerkes aus Stahlbeton nach einem der Ansprüche 1 bis 6, **dadurch gekennzeichnet, dass** Messparameter, wie beispielsweise Messzeitpläne, Messdauern, Messprotokolle über die Cloud oder die externe Auswerteeinheit an das Gateway (6) und/oder Router (8) und/oder Endgeräte (2) verteilt werden bzw. diese aktualisiert werden.

8. Stahlbetonbauwerküberwachungssystem (1) zur Durchführung des Verfahrens nach einem der Ansprüche 1 bis 7, umfassend ein Mesh-Netzwerk mit einer Mehrzahl von Endgeräten (2), wobei ein Endgerät (2) mindestens ein Sensorelement umfasst oder mit mindestens einem Sensorelement signaltechnisch verbunden ist,
und mindestens einem Gateway (4), wobei die Endgeräte (2) signaltechnisch direkt oder indirekt mit dem Gateway (4) verbunden sind und das Gateway zur Kommunikation mit einer Cloud (6) oder externen Auswerteeinheit ausgebildet ist.

9. Stahlbetonbauwerküberwachungssystem (1) nach Anspruch 8, **dadurch gekennzeichnet, dass** eine Anzahl von Routern (8) vorgesehen ist, die signal- und kommunikationstechnisch zwischen den einzelnen Endgeräten und dem Gateway angeordnet sind.

10. Stahlbetonbauwerküberwachungssystem (1) nach einem der Ansprüche 8 oder 9, **dadurch gekennzeichnet, dass** zumindest einzelne Sensorelemente aus der Gruppe von Beschleunigungsmesser (26), GNSS-Sensoren (18), Dehnungsmessstreifen (20), Betonzustandssensoren (22), insbesondere Anodenleiter, Sensoren zur Messung des elektrischen Widerstandes, Karbonatisierungssensor (36), Referenzelektrode (52) und Riss- und Fugensensoren (34) sind.

11. Stahlbetonbauwerküberwachungssystem (1) nach Anspruch 10 in Kombination mit einer Referenzelektrode (52), **dadurch gekennzeichnet, dass** die Referenzelektrode (52) eine Kammer (56) umfasst, die zumindest teilweise mit einem Elektrolyt gefüllt ist, und eine Elektrode (60) umfasst, die im Betriebszustand in die Kammer (56) und das Elektrolyt eintaucht, wobei zumindest an einer Seite der Kammer (56) ein leitfähiges Kontaktelement (62) vorgesehen ist, welches in direkten Kontakt mit dem Elektrolyt steht und welches eine Kontaktoberfläche aufweist, wobei das Kontaktelement (56) als elastischer Feststoff ausgebildet ist.
